# EUROPEAN PATENT APPLICATION

(11) **EP 3 957 992 A1**
(43) Date of publication of application: **23.02.2022**
(21) Application number: 20191666.5
(22) Date of filing: 19.08.2020
(51) Int. Cl.: G01N 33/50

(54) **METHOD FOR DETERMINATION OF BASOPHIL ACTIVATION AND KITS THEREFORE**

(71) Applicant: Forschungszentrum Borstel, Leibniz Lungenzentrum, 23845 Borstel (DE)
(72) Inventor: BEHRENDS, Jochen, 23847 Lasbek-Gut (DE); SCHWAGER, Christian, 23845 Borstel (DE); JAPPE, Uta, 23843 Bad Oldesloe (DE); SCHOLZEN, Thomas, 23843 Neritz (DE); HEIN, Martina, 23795 Bad Segeberg (DE)
(74) Representative: Gramm, Lins & Partner Patent- und Rechtsanwälte PartGmbB

(57) **Abstract**

The present invention relates in a first aspect to a cytometric based *in vitro* method for the determination of basophil activation in a sample obtained from a subject which method can be conducted automatically and preferably in a computer implemented method for fast and reliable determination of basophil activation. In a further aspect, a test kit or a kit of parts for determining of basophil activation in a subject is provided. Said method and test kit or kit of parts is particularly useful for determining basophil activation after contacting the cell containing sample with a test substance. The use includes the determination of a subject's basophil activation whereby said subject suffers from an allergy or an autoimmune disease.

## Description

The present invention relates in a first aspect to a cytometric based *in vitro* method for the determination of basophil activation in a sample obtained from a subject which method can be conducted automatically and preferably in a computer implemented method for fast and reliable determination of basophil activation. In a further aspect, a test kit or a kit of parts for determining of basophil activation in a subject is provided. Said method and test kit or kit of parts is particularly useful for determining basophil activation after contacting a cell containing sample with a test substance. The use includes the determination of a subject's basophil activation whereby said subject suffers from an allergy or an autoimmune disease.

### Prior art

In the past century, allergic disorders have emerged from a rare disease to a most common chronic disease in Western countries, affecting approximately 30% of the population. Moreover, their prevalence is still rising and will become a problem for the population of the world. Actual prediction from the European Academy of Allergy and Clinical Immunology estimates a rise to 50% for the European population until 2025. Although effective immunotherapies are readily available for the majority of allergic patients, a number of individuals are not adequately treated due to misdiagnosis of their disease. Misdiagnosis is often related to the use of diagnostic tests and the incorporated allergens. At present, the common routine allergy tests are skin prick test (SPT) and serological IgE analysis. Both systems are based on determining the existence of IgE antibodies against the allergen tested, however, the systems are not suitable for assessing the actual biological activity and the status of allergy. In other words, while the routine diagnostic tests show sensitization against the allergen, the activity of the subject tested against said allergen, namely, the allergy itself is not examined. Further, interpretation of positive test results is often complicated due to the existence of highly cross-reactive but clinically irrelevant allergens. That is, the above describe routine tests do not prove the presence of the active allergy, and the allergic status as the IgE-mediated release of mediators such as histamine can be prevented by blocking antibodies that are not addressed by the classical allergy tests. Therefore, the clarification of the actual status of allergy of patients with equivocal history and routine test is occasionally conducted by time-consuming and potentially risky allergen challenge tests, which are the current gold standard of allergy diagnosis. This type of provocation test is also life threatening in case of some allergen sources like insect venoms and peanuts, and, therefore, a laborious, time-consuming work often with a hospital stay is required for conducting these types of tests. In recent years a diagnostic test that assesses effector cells involved in the release of allergen mediators such as histamine has gained much attention, the basophil activation test (BAT). The BAT mimics the allergic reaction *in vitro* thereby discriminating between allergic patients and non-allergic individuals as well as sensitized-only subjects, which are individuals with sensitization but without symptoms. This *in vitro* test has many advantages, for example, it has a good safety profile as well as high sensitivity and specificity and is capable of predicting the severity of an allergic reaction. The BAT takes into account the effects of protective antibodies as negative regulators of an allergic reaction. These characteristics make the BAT superior to the SPT and IgE detection tests described above. The basis for the BAT is IgE-mediated activation of basophils in whole blood, which has the advantage of also taking the blocking antibodies into account.

Therefore, BAT may replace the SPT and the IgE detection tests but also the time-consuming and life-threatening allergen challenge tests in the future. However, at present, BAT is not applied in broader clinical application due to high costs, complexity of the analysis as well as logistical issues like the limited time span between blood donation and sample measurement.

Up to now, four different BAT kits are available on the market; two of them are already certified for clinical routine. All of them are single analysis kits that have to be performed manually in tubes, thus restricting the use in high routine diagnostics. Moreover, sensitivity and specificity are not satisfactorily resulting in high cut offs for positivity. Further, various strategies have been suggested for conducting the BAT protocols based on different expression profiles of molecules. However, the minimalistic selection of identification markers including activation markers is a pitfall that often causes high cut offs, due to the contamination of the basophil population with other cells.

At present, beside identification markers, two proteins are widely accepted as activation markers for basophils, namely, the CD63 molecule and the ectoenzyme CD203c.

EP 2 037 269 B1 refers to an allergy test based on cytometric analysis. This method is based on determining changes of the mean or median fluorescence intensity (MFI) of the IgE antibodies bound to the Fc_{ε}-receptor on the cell surface of basophils and CD63 antibodies bound to the CD63 antigen also present on the cell surface of basophils after activation based on a specific activation index of the MFI of CD63 and IgE. Further, the method described therein relies on the determining CCR3 expression as selection marker for basophils. In addition, an *in vitro* allergy test method is provided based on human whole blood samples. However, this method suffers from the fact that CCR3 is used as sole selection marker of basophils while stimulation of the cells is associated with a down regulation of the expression of CCR3. Hence, a CCR3-based selection becomes difficult and a contamination with other cells is possible.

Recently, a BAT protocol has been published (Schwager C., et al., J Allergy Clin Immunol, 2017, 140(5), 1331 - 1338), showing both, high sensitivity and specificity as well as a low cut off for positivity in peanut allergic patients. A main problem of this BAT protocol disclosed therein is that it is currently too expensive and complex to be used in a routine diagnostic setting.

Hence, there is a need for providing a more accurate *in vitro* test that allows high through-put determination of allergic patients and is useful in routine diagnostic settings while maintaining the diagnostic performance.

### Brief description of the present invention

In a first aspect, the present invention provides a method for the determination of basophil activation in a sample obtained from a subject comprising
a. providing a sample of said subject;
b. optionally, treating/stimulating the sample with a test substance;
c. adding a mixture of antibodies each labelled with a different fluorochrome, said mixture containing the labelled antibodies anti-CD63, anti-CD203c and anti-FcεRIα, and incubating said mixture with said sample;
d. measuring the labelled sample obtained in step b) by flow cytometric based measurement;
e. determining basophil activation based on the analysis of the measured samples and obtained data by flow cytometry-based measurement whereby the analysis is based on the following:
   i. excluding doublets, in particular by analyzing forward scatter area versus forward scatter height, and gating of single cells;
   ii. analyzing the cells gated in i) and gating basophils being double positive for FcεRIα and CD203c;
   iii. analyzing the basophils gated in ii) on forward scatter area versus side scatter area and gating of basophils;
   iv. analyzing the basophils selected in iii) for expression of CD203c versus expression of CD63 and determining the percentage of basophils expressing both CD203c and CD63 representing activated basophils.

In a further aspect, the present invention relates to a computer implemented method for the determination of basophil activation in samples obtained from a subject comprising the steps of:
a. obtaining data of measured parameters by flow cytometry including the following: forward scatter area, forward scatter height, side scatter area, fluorescence of the labelled, basophil-bound antibody anti-CD203c, fluorescence of the labelled, basophil-bound antibody anti-FcεRIα, fluorescence of the labelled, basophil-bound antibody anti-CD63 in a stimulated or non-stimulated sample of a subject;
b. computing the data obtained as follows:
   i. doublet exclusion, in particular, based on forward scatter area versus forward scatter height;
   ii. analyzing the cells gated in i) and gating basophils being double positive for FcεRIα and CD203c
   iii. analyzing the basophils gated in ii) on forward scatter area versus side scatter area and gating of basophils;
   iv. analyzing the basophils selected in iii) for expression of CD203c versus expression of CD63 and determining the percentage of basophils expressing both CD203c and CD63, representing activated basophils;
c. identifying the percentage or absolute number of basophil activation in said sample of said subject;
d. optionally further comprising the step of showing the data on an output unit, in particular, showing the percentage of basophil activation;
e. optionally, quality control of the samples based on at least one of i) minimum amount of basophils to be analyzed, ii) minimum background activation of PBS samples (like below 5%), iii) minimum amount of all events (like above 10,000, and iv) maximum amount of doublets (like below 20% of all events).

Moreover, a computer related medium or computer program product having computer executable instructions for performing the steps of the computer implemented method according to the present invention.

In addition, a test kit or a kit of parts for determining the basophil activation in a subject is provided, said kit comprising anti-CD203c, anti-FcεRIα and anti-CD63 antibodies, each labelled with a distinct fluorochrome, optionally lysing buffer for erythrocytes and optionally a washing buffer as well as instructions to use said test kit or kit of parts in a method according to the present invention. The test kit can be used in determination of basophil activation, like basophil activation induced *in vitro* by test substances based on flow cytometric measurement. In addition, the test kit or the kit of parts is useful in determining whether a said subject has an allergy against a test substance and/or is suffering from an autoimmune disease.

### Brief description of the drawings

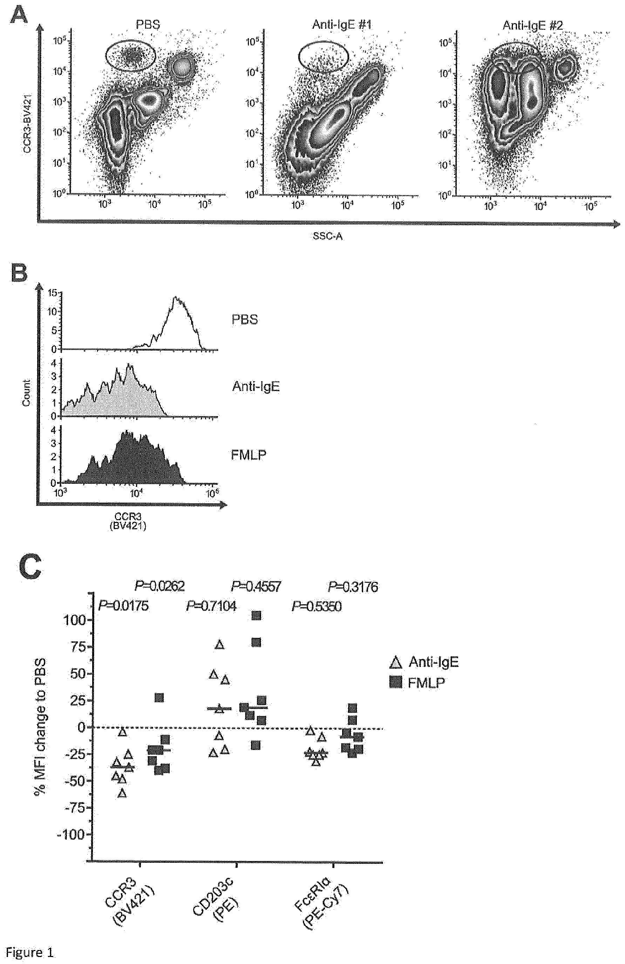

**Figure 1** Analysis of stimulation-induced shift of basophil markers. (A) Identification of basophils by applying the CCR3-gating strategy to PBS samples (control) and two selected anti-lgE stimulated samples (anti-lgE #1 and anti-lgE #2) shows difficulties in adapting the recommended gating strategy (especially anti-lgE #2): distinction between basophils and other blood cells is impossible. (**B**) Histograms of CCR3-expression: unstimulated PBS samples and samples stimulated with anti-lgE and fMLP. (**C**) Changes of the median fluorescence intensities (MFIs) upon stimulation with anti-lgE or fMLP for the basophil markers CCR3, CD203c and FcεRIα. Black bars indicate medians of the group. Statistical significances were determined using the Mann-Whitney U test: *^{*}P* <0.05.

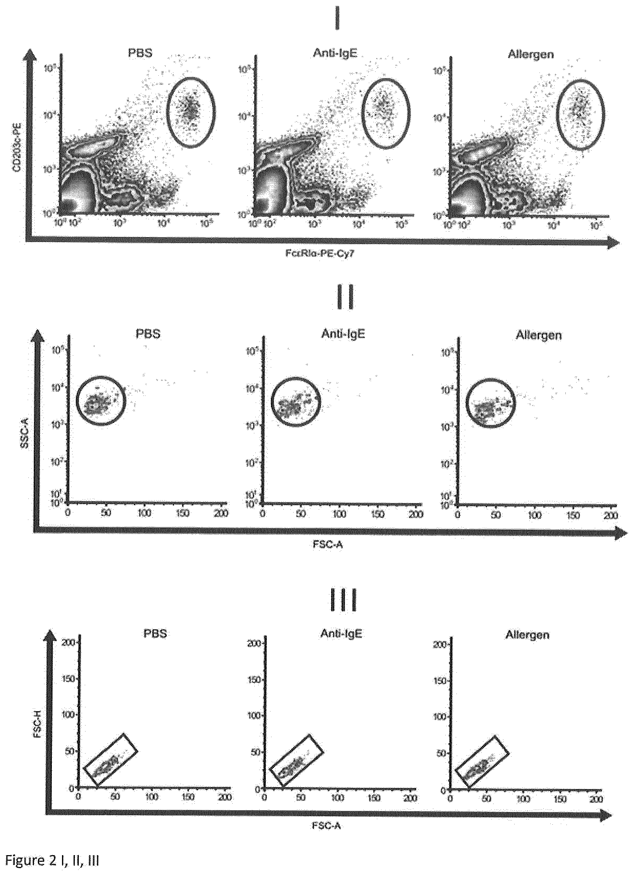

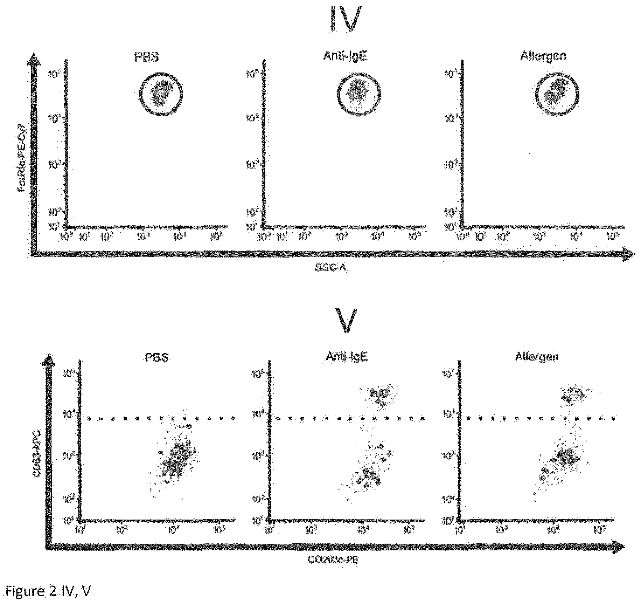

**Figure 2** Detailed overview of the new two identification marker-based gating strategy associated with the 3 ab protocol according to the present invention. Exemplary gating of basophils of a peanut-allergic patient stimulated with PBS, anti-lgE and peanut oleosins (allergen). (I) Identification of FcεRIα/CD203c positive basophils, (II) debris exclusion, (III) doublet exclusion, (IV) dendritic cell exclusion and (V) evaluation of the basophil activation by CD63.

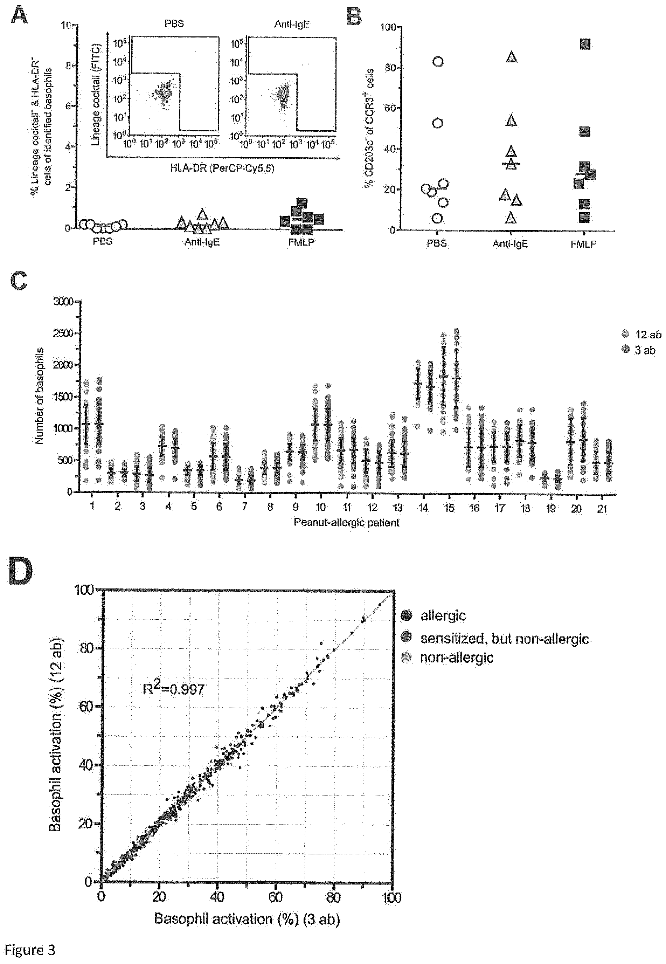

**Figure 3** Comparison of basophil gating strategies. (**A**) Analysis of the identified basophil population after application of the two identification marker-based gating strategy according to the present invention for contaminations with other cells using anti-lineage cocktail antibodies (anti-CD3, anti-CD14, anti-CD16, anti-CD19, anti-CD20, anti-CD56) and anti-HLA-DR antibodies in samples stimulated with PBS, fMLP and anti-IgE, respectively. Cells in the box of the inserts (representative gating) are considered as impurities. (**B**) Analysis of the identified basophil population (CCR3^{high}/SSC^{low}, see gating fig. 1A) using the CCR3 protocol by gating for the basophil identification marker CD203c. The number of cells, which do not express CD203c, is presented, indicating contaminations with non-basophil cells. (**C**) Comparison of the number of identified basophils after application of either the 12 ab protocol of the prior art or 3 ab protocol according to the present invention. Means and standard deviations are indicated. (**D**) Comparison of the basophil activation level (% CD63 expression of identified basophils; PBS, anti-lgE and fMLP samples) between the 12 ab and the 3 ab protocol according to the present invention assessing 1,104 individual samples. Black bars indicate medians of the group.

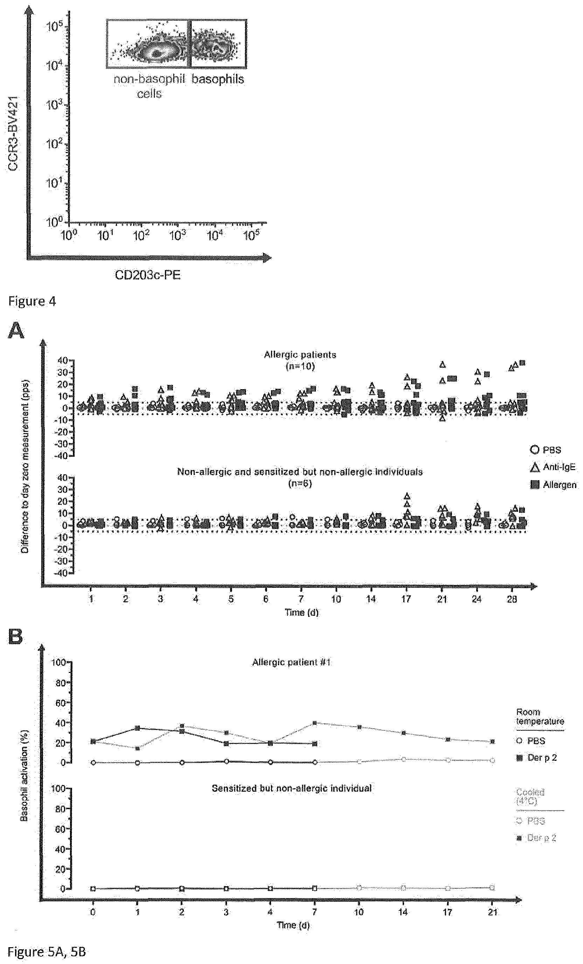

**Figure 4** Analysis of the basophil purity using the CCR3-protocol. Gated basophils (CCR3^{high}/SSC^{low}) were further analyzed for CD203c to identify basophils (CD203c positive) and non-basophil cells (CD203c negative).

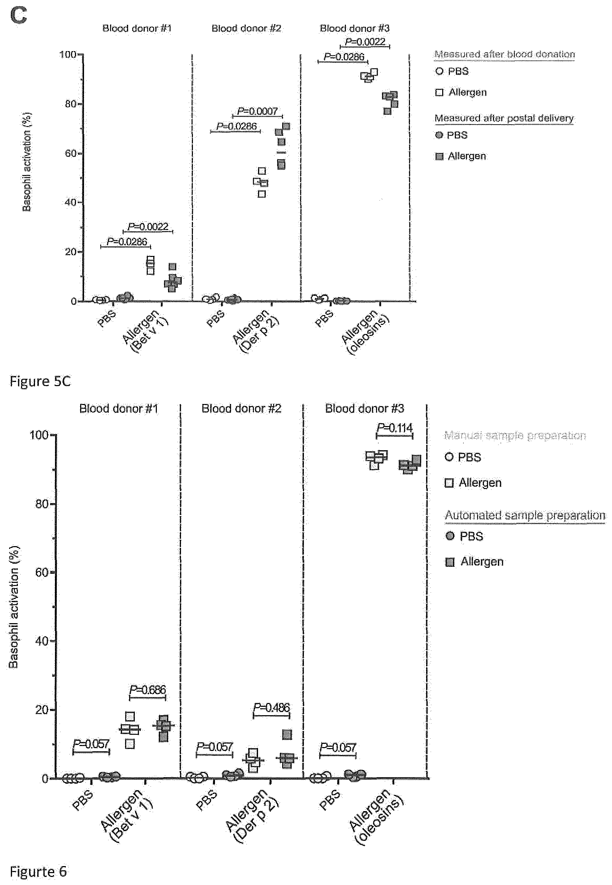

**Figure 5** Evaluation of the BAT after storage of prepared samples and whole blood. (**A**) Progression of the basophil activation (% of CD63 expressing basophils) for prepared samples (PBS: circle, anti-lgE: triangle, allergen: square) stored at 4°C over a time period of 28 days. Data show changes (in percentage) of activation in comparison to direct measurement at day 0. (**B**) Progression of the basophil activation (% CD63⁺) for whole blood samples (PBS: circle, Der p 2: square) from a house dust mite-allergic and a sensitized but non-allergic individual over a period of 21 days. Blood was stored at 4°C (grey) or RT (darkgrey), then stimulated (PBS: circle, Allergen: square) and prepared for analysis at each of the given days. (C) BAT results (% CD63⁺) of whole blood samples of 3 allergic donors (Bet v 1 (birch pollen major allergen), Der p 2 (house dust mite major allergen), oleosins (peanut marker allergen for severe reactions)) at the day of blood donation and preparation (grey) as well as after postal delivery and preparation (darkgrey). Bars indicate medians of the group.
**Figure 6** Comparison of automated and manual sample preparation and measurement. Blood from 3 donors was subjected in parallel to manual and automated preparation and the basophil activation analyzed thereafter using the 3 ab protocol of the present invention. Automated analysis was performed on a MQ10 and analysis of manually prepared samples on an LSRII. Bar indicates the median for each group. There was no statistically significant difference between the results of both methods (Mann-Whitney *U* test).

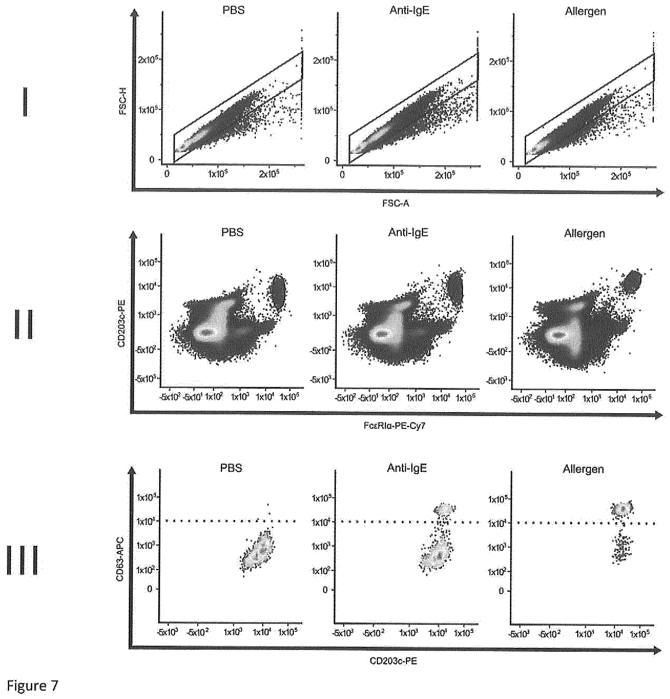

**Figure 7** Overview of the 3 ab automated gating strategy (R analysis). (I) After doublet exclusion (FSC-A vs. FSC-H), basophils are identified as (II) CD203c⁺/FcεRIα⁺ cells and (III) analyzed for activation status by evaluation of the CD63⁺ fraction. The auto-gating algorithm performs an adjustment of the individual gates for each sample.

### Detailed description of the invention

In the first aspect, the present invention relates to a cytometric based method for the determination of basophil activation in a sample obtained from a subject comprising
a. providing a sample of said subject;
b. optionally, treating/stimulating the sample with a test substance;
c. adding a mixture of antibodies each labelled with a different fluorochrome, said mixture containing the labelled antibodies anti-CD63, anti-CD203c and anti-FcεRIα, and incubating said mixture with said sample;
d. measuring the labelled sample obtained in step b) by flow cytometric based measurement;
e. determining basophil activation based on the analysis of the measured samples and obtained data by flow cytometry based measurement
   whereby the analysis is based on the following:
   i. excluding doublets, in particular by analyzing forward scatter area versus forward scatter height, and gating of single cells;
   ii. analyzing the cells gated in i) and gating basophils being double positive for FcεRIα and CD203c;
   iii. analyzing the basophils gated in ii) on forward scatter area versus side scatter area and gating of basophils;
   iv. analyzing the basophils selected in iii) for expression of CD203c versus expression of CD63 and determining the percentage of basophils expressing both CD203c and CD63 representing activated basophils.

As used herein the term "double positive for" refers to basophils or other cells where the respective molecules are expressed and detected by respective antibodies against said molecule. E. g. a cell, like a basophil, is referred to as double positive for CD203c and FcεRIα, if the cell is expressing both molecules, and (in addition) the corresponding molecules have been recognized/bound by fluorescent-labelled antibodies, so that the resulting fluorescence is above a cut off value or exceeds a given threshold.

The terms "fluorophore" and "fluorochrome" are used herein interchangeably and synonymously unless otherwise indicated.

The aim of the present invention is to provide a method for automating the work process and the data analysis, in order to improve the usability of the BAT in routine laboratories.

The sample of said subject is obtained in advance. Typically, the sample is selected from fluid or enzymatically digested tissue for serial cell suspension derived from said subject. In an embodiment the sample is collected from sputum, blood, including whole blood, saliva, nasal secretion, and urine, preferably the sample is whole blood. In particular, the sample is whole blood comprising anticoagulation compounds. The whole blood may be whole blood obtained from the subject or pre-treated whole blood like purified whole blood. As noted, the whole blood may contain anticoagulation compounds. Typical anticoagulation compounds include EDTA, citrate, and heparin.

Moreover, in an embodiment, the whole blood is treated in advance for lysis of erythrocytes present in said sample when obtained from the subject accordingly.

The sample may be treated or stimulated with a test substance in advance. For example, in order to determine basophil activation in the context of allergies and/or autoimmune diseases, these test substances used for treating and potentially stimulating a sample could be allergens or compounds relevant in autoimmune disease. Treating or stimulating the sample means that cells present in said sample including basophils are brought into contact with the test substance accordingly. The test substance may be a mitogen, an allergen, a protein or peptide, a protein or peptide allergen, a group or mixture of protein or peptide allergens, a non-proteinaceous allergen, a low molecular weight allergen, a low molecular weight drug substance or a hapten. These kinds of test substances include test substances coupled or bound to carrier molecules known to the skilled person, like bi- or multifunctionalized compounds such as chemical linkers, peptides, proteins, these include NHS-ester, alkines, amines, biotin, oxidized carbohydrates, carboxyles, sulfhydryles, streptavidin, amin-amin-crosslinker, sulfhydryl-carbohydrate-crosslinker, sulfhydryl-sulfhydryl-cross-linker, amin-sulfhydryl-crosslinker, carboxyl-amin-crosslinker, PEG, azides.

The allergen may be a food additive, preferably a food additive selected from a group consisting of emulsifiers, food colorants, food preservatives, food finishes and antioxidants. The allergen may also be a protein or mixture of proteins or a protein extract from a biological source or a drug. Drugs include Nonsteroidal anti-inflammatory drugs like Aspirin, paracetamol, metamizol, diclofenac, naproxen and other drugs of the group of NSAIDs; Biologica including Cetuximab, Adalinumab, Omalizumab, Benralizumab, Etanercept, Infliximab, Golimumab, Belimumab,Rituximab, Tocilizumab and others; Antibiotics including Betalaktame (benzylpenicilloyl-polylysine (PPL), penicillin, ampicillin, amoxicillin, cephalosporins); and muscle relaxans including suxamethonium, gallamine, vecuronium, and pancuronium. Extracts from biological sources inducing allergy include the group of insect venoms, foods, including fruits, vegetables, seeds, legumes, nuts, spices, fish, shellfish, mollusks, foal, meat and milk. In addition, tree pollens, grass pollens, weed pollens, epidermal and animal proteins, dust and storage mites, insects, parasites, microorganisms and house dust may be mentioned as source for the allergen which is used for the test substance.

The test substance is added in suitable amounts to the sample. Suitable amounts are known to the skilled person, and typically, the allergen is added in an amount of 0.1 ng/ml to 10 mg/ml.

To analyze a sample that either has been pre-treated with a test substance or not, a mixture of antibodies is added. Each of these antibodies is labelled with a different fluorochrome. The mixture of antibodies contains labelled antibodies, namely, anti-CD63, anti-CD203c and anti- FcεRIα.

The antibodies may be commercially available antibodies of different species. Typically, the antibodies are monoclonal antibodies labelled with a fluorochrome. However, it is also possible, that the antibodies are not directly labelled, but rather recognized by a labelled secondary antibody directed against the primary antibody, namely, any one of the antibodies of anti-CD63, anti-CD203c and anti-FcεRIα.

The cells present in the sample are incubated with said mixture of antibodies. Typically, after incubation for a sufficient period of time to allow binding of the antibodies to the specific target molecules, the cells present in the sample are washed with a suitable washing buffer. Suitable washing buffers are preferably selected from any kind of washing buffers known from prior art to neutralize the effect of the lysing reagent and to eliminate disturbing factors and cell debris after erythrocyte lysis:
After labelling, the labelled sample is measured by flow cytometric based measurement. The skilled person is well aware of suitable apparatus allowing flow cytometric based measurements. The flow cytometric based measurements include suitable sources of light, typically, lasers for excitation of the fluorescent dyes attached to the antibodies. Moreover, suitable detectors are present for measuring the different parameters. Typically, the measured parameters by flow cytometry include at least the following: forward scatter signal, including the height and area thereof, a side scatter signal, including the area and height thereof, the fluorescence of the labelled antibodies. The detectors allow differentiating and specifically measuring fluorescence emitted by the different fluorochromes. The detectors include suitable optical devices for specific measurement.

The data obtained from the detectors are further processed. Namely, the basophil activation is determined based on the analysis of the measured samples and the data obtained by flow cytometric based measurement whereby the analysis is based on several steps including in a first step of, exclusion of doublets of cells. Typically, doublets of cells can be excluded by analyzing the data of the forward scatter area vs. forward scatter height. Only single cells are gated and analyzed further in the next step, where these single cells are then analyzed for the expression of FcεRIα and CD203c. Activated basophils are expressing both molecules. Thus, single cells being double positive for these two antigens, namely FcεRIα and CD203c, are gated as cells containing activated basophils.

These basophils gated for double positivity are then further analyzed based on the forward scatter area vs. the side scatter area, in order to differentiate the single cells obtained according to size and granularity. Since basophils are granulated cells, it is possible to exclude non-granulated cells based on the side scatter area.

These gated basophils obtained from the step above are then finally analyzed for the activation status of the basophils, i.e. their expression of the CD203c and CD63. Cells expressing both, CD203c and CD63, are regarded as activated basophils and, consequently, it is possible to determine the percentage of basophil activation, i.e. the proportion of activated basophils compared to non-double positive cells.

In an embodiment, a further step is included wherein the basophils obtained in step iii), namely by gating on forward scatter area vs. side scatter area, are further analyzed on the side scatter area vs. the measured fluorescence of the antibody against FcεRIα. FcεRIα positive basophils are gated and further analyzed for the expression of CD203c vs. expression of CD63.

In an embodiment of the present invention, the method is a computer implemented method for the determination of basophil activation in samples obtained from a subject comprising steps of
a. obtaining data of measured parameters by flow cytometry including the following: forward scatter area, forward scatter height, side scatter area, fluorescence of the labelled, basophil-bound antibody anti-CD203c, fluorescence of the labelled, basophil-bound antibody anti-FcεRIα, fluorescence of the labelled, basophil-bound antibody anti-CD63 in an stimulated or non-stimulated sample of a subject;
b. computing the data obtained as follows:
   i. doublet exclusion, in particular, based on forward scatter area versus forward scatter height;
   ii. analyzing the cells gated in i) and gating basophils being double positive for FcεRIα and CD203c
   iii. analyzing the basophils gated in ii) on forward scatter area versus side scatter area and gating of basophils;
   iv. analyzing the basophils selected in iii) for expression of CD203c versus expression of CD63 and determining the percentage of basophils expressing both CD203c and CD63, representing activated basophils;
c. identifying the percentage or absolute number of basophil activation in said sample of said subject;
d. optionally further comprising the step of showing the data on an output unit, in particular, showing the percentage of basophil activation;
optionally, quality control of the samples based on at least one of i) minimum amount of basophils to be analyzed, ii) minimum background activation of PBS samples (like below 5%), iii) minimum amount of all events (like above 10,000, and iv) maximum amount of doublets (like below 20% of all events).

That is, the data of measured parameters obtained by flow cytometry for a given sample include data of the forward scatter area, the forward scatter height, the side scatter area, the measured fluorescence of the labelled antibodies against CD203c, FcεRIα, and CD63. The sample may be a stimulated, i.e. a pretreated sample or a non-stimulated sample of the subject, whereby the basophil activation should be determined.

The data is computed in a first step wherein doublets or other types of unusual size distribution including cell debris are excluded by appropriate gating. Thus, only single cells are further analyzed. In the next step, the data are computed to gate basophils which are double positive for FcεRIα and CD203c.

The double positive FcεRIα and CD203c basophils are further analyzed on the forward scatter area vs. the side scatter area, in order to separate single cell basophils from cell debris and doublets. Gated single cell basophils are then analyzed for the expression of CD203c and CD63 as markers for basophil activation. The amount of basophils expressing both molecules, namely CD203c and CD63, is determined. This fraction represents the activated basophils and may be expressed as a percentage of all basophils or an absolute number.

In an embodiment, the data, i.e. the percentage of all basophils or the absolute number of activated basophils, preferentially the percentage of basophil activation, are displayed on an output unit.

The computer implemented method as well as the method of cytometric based *in vitro* determination of basophil activation is described herein and may optionally contain a quality control of the samples. Said quality control of the samples may be based on at least one of (i) the minimum amount of basophils being analyzed and/or (ii) the minimum background activation of the PBS samples and/or (iii) the minimal amount of all events and/or (iv) the maximum amount of doublets. For example in case of the minimal background activation of PBS samples, the background should be below 5%, preferentially below 3%. In case of the minimal amount of all events, all events should be at least 10,000 events preferentially above 20,000 events. Moreover in case of the maximal amount of doublets, the maximum amount of doublets should be below 20% of all events, preferentially below 10% of all events.

The skilled person is well aware of determining the respective control values accordingly.

In an embodiment of said computer implemented method, the determination of the basophil activation is a method wherein the basophils are activated or induced by test substances in a sample derived from a subject, in particular, wherein the test substance is an allergen. Of course, all of the test substances mentioned above may be applied accordingly.

The present invention further relates to a computer medium or computer program product having computer executable instructions for performing the steps as identified in a method according to the present invention.

Moreover, a test kit or a kit of parts is provided. Said test kit or kit of parts is for determining of basophil activation in a sample derived from a subject. The kit or kit of parts comprises anti-CD203c, anti-FcεRIα and anti-CD63 antibodies, each labelled with a distinct fluorochrome as well as instructions on how to use said test kit or kit of parts in a method according to the present invention. The test kit or kit of parts may further comprise lysis buffer for erythrocytes and, optionally, washing buffer. Moreover, the test kit or kit of parts may contain one or more test substances used for activating the basophils.

The test kit or the kit of parts according to the present invention is useful for determining basophil activation by flow cytometric measurement. In an embodiment, basophil activation is induced by a test substance *in vitro.*

In a further embodiment, the test kit or kit of parts is useful for determining basophil activation in a sample derived from a subject having an allergy against the test substance, whereby the test substance is an allergen and whereby said allergy is determined based on determination of basophil activation in the presence of the test substance. Alternatively, the test kit or kit of parts is useful in determining whether a subject suffers from an autoimmune disease.

In addition, the method according to the present invention is a method wherein the sample processing and measurement are conducted automatically using a flow cytometer with integrated robotic sample handling and manipulation. In a further embodiment, the basophil activation is determined using the computer implemented method according to the present invention.

A further embodiment according to the present invention relates to a method wherein the blood sample to be analyzed has been stored at room temperature for up to 7 days or at 4°C for up to 17 days before stimulation, processing and measurement or alternatively, wherein the stimulated sample has been stored in the dark at 4°C for up to 28 days before determination of the basophil activation.

Moreover, in another embodiment, the method according to the present invention is a method wherein step e) is conducted by computer implemented analysis.

As noted, the test substance may be an allergen, in particular, selected from a group of inhalant allergens, food allergens, insect allergens and pharmaceutical based allergens, including peanut allergens, like peanut oleosins, like Ara h 14, Ara h15, peanut defensins, Der p 2, Bet v 1, Ara h 1, Ara h 2, Ara h 6, Ara h 8..

Further, the allergen may be an extract from sesame, lupines, peanut, hazelnut, walnut, almonds, pecans, pistachios, and Brazil nuts, chestnuts, acorns, soybean, egg, cow's milk, wheat, celery, birch pollen, timothy, mugwort, house dust mite, cat dander, dog dander, *alternaria* and other mold fungus species, carotte, codfish.

In addition, contact allergens, like latex, nickel, etc. may be used as test substances as well.

In an embodiment of the method according to the present invention, the flow cytometry is conducted using at least two phases for examination of the fluorochrome label on the antibodies. In an embodiment, the first laser for forward and side scatter measurement as well as the identification of the basophils, and a second laser for the measure of the basophil activation status. Namely, the identification of the basophils is conducted using the expression of the molecules FcεRIα and CD203c, while the basophil activation status is determined inter alia using the expression of CD63.

In a further embodiment, the fluorochromes are selected for low spillover, in particular, the fluorochromes used with the first laser are PE and PE-Cy7, and allophycocyanin (APC) with the second laser.

The skilled person is well aware of suitable laser and laser combinations appropriate for the methods according to the present invention. In particular, the skilled person is well aware of suitable laser combinations and optical components for measuring the respective parameters including the fluorescence with a low spillover, namely, with low overlapping wavelengths of the fluorescence dyes.

The present invention will be described further by way of examples without limiting the same thereto.

### Examples

### Material and Methods

### Study populations

Allergic, sensitized but asymptomatic and non-allergic individuals were recruited in the allergy outpatient clinics of Borstel and Luebeck. After thorough documentation of clinical history, the status of the study population was confirmed by serology and/or basophil activation test.

### Flow cytometric instruments and antibodies

Measurements were conducted on an LSRII instrument (BD Biosciences, San Jose, Calif), of which 3 lasers and 7 (12 ab gating strategy) or 3 (3 ab gating strategy) detection bandpass (BP) filters were used for manual data acquisition (configurations listed in table S4). Automatic sample preparation and measurement were performed on a MACSQuant10 (Miltenyi Biotec, Bergisch Gladbach, Germany). For the inter-laboratory testing 10 further flow cytometers (BD Biosciences) were involved: 3x FACSCantoll, 2x LSRII, 2x Fortessa, 1x Symphony, 1x FACSLyric and 1x FACSCalibur.

**Table 1 Antibodies used in the BAT experiments**

| **Antibody** | **Clone** | **Fluorochrome** | **Manufacturer** |
|---|---|---|---|
| **Anti-human CD45** | HI30 | BV510 | Biolegend (Fell, Germany) |
| **Anti-human CD63** | H5C6 | APC | Biolegend (Fell, Germany) |
| **Anti-human CD123** | 6H6 | BV421 | Biolegend (Fell, Germany) |
| **Anti-human CD203c** | NP4D6 | PE | Biolegend (Fell, Germany) |
| **Anti-human FcεRIα** | AER-37 | PE-Cy7 | Biolegend (Fell, Germany) |
| **Anti-human HLA-DR** | L234 | PerCP-Cy5.5 | Biolegend (Fell, Germany) |
| **Anti-human lineage cocktail** | | FITC | Biolegend (Fell, Germany) |
| **Anti-human CD3** | UCHT1 | | |
| **Anti-human CD14** | HCD14 | | |
| **Anti-human CD16** | 3G8 | | |
| **Anti-human CD19** | HIB19 | | |
| **Anti-human CD20** | 2H7 | | |
| **Anti-human CD56** | HCD56 | | |

### Allergens and stimulants

The following allergens were either isolated and purified from natural (n) sources or recombinantly expressed (r) in *E. coli* BL21 (DE3): peanut oleosins (n), peanut defensins (n), Der p 2 (r), Der p 2 (n), Bet v 1 (n), Ara h 8 (r), Ara h 14 (r), Ara h 15 (r). The identity of each allergen was verified on a QExactive hybrid quadrupole-orbitrap mass spectrometer (Thermo Scientific, Waltham, MA). Further allergens were purchased from Indoor Biotechnology Ltd. (Cardiff, UK): Ara h 1 (n), Ara h 2 (n), Ara h 6 (n). The stimulants formyl-methionyl-leucyl phenylalanine (fMLP 1 µM, Sigma-Aldrich, Steinheim, Germany) and anti-lgE (1:1 mixture of goat anti-human IgE (1 µg/mL, Sigma-Aldrich, Steinheim, Germany) and goat anti-human IgE (1 µg/mL, Abcam, Cambridge, UK) were used as positive controls for the BAT, PBS buffer as negative control.

### Basophil activation test

The 12 antibody basophil activation test (12 ab BAT) and the identification of basophils (gating) was performed as reported previously (Schwager et al. [2017] J. Allerg. Clin Immunol. 140:1331-1338). For the evaluation of the 3 antibody gating strategy (3 ab BAT), the data acquired for the 12 ab BAT were taken and cells gated. Analysis was conducted with the same fixed quadrant for the final CD203c vs. CD63 gating for both, the 12 ab and the 3 ab evaluation. The work-up of the BAT with CCR3 was the same as for the 12 ab BAT with the exception that a reduced set of four anti-human antibodies (Biolegend, San Diego, CA) was used (CCR3-BV421, CD45-BV510, CD203c-PE and CD63-APC). Basophils were identified and their activation status analyzed according to the gating strategy shown in Figure 2. The total numbers of detected basophils were recorded. Data analysis and statistics were conducted using GraphPad Prism software package (version 7, GraphPad Software, San Diego, CA).

### Automation process

For the automation process, lysis buffer was prepared first, and the tubing of the storage solution was put into the lysis buffer reservoir. After that, the flow cytometer was flushed extensively with lysis buffer. Furthermore, the anti-human antibody mixture was prepared (3 ab mix: FcεRIα-PE/Cy7, CD203c-PE and CD63-APC) in a dark vial which was placed on the reagent rack 4 of the MACSQuant. Stimulants were then placed in a 96-well deep well plate (Sarstedt AG & Co. KG, Nümbrecht, Germany) and mixed manually with 150 µl freshly heparinized blood. Thereafter, the plate was mounted onto the 96-well rack (chill 96) of the MACSQuant. Next, the run was started with following parameters: flow rate (high), mix sample (medium), mode (fast), uptake volume (450 µl), sample volume (150 µl). After 30 min of incubation with the stimulants, 50 µl of antibody mixture was automatically added. 20 min later, 400 µl of lysis buffer was added by the instrument. 25 min later, 600 µl of MACS running buffer was added, and the samples were automatically analyzed by the instrument.

### Time series measurement: one time sample work up and continuous analysis

Heparinized whole blood of 15 study patients (10 allergic patients, 3 sensitized but non-allergic subject and 3 non-allergic individual) was stimulated (PBS, anti-lgE, or allergen, 5,000 ng/ml) and prepared for measurement according to the published standard protocol (Schwager et al. [2017] J. Allerg. Clin Immunol. 140:1331-1338). The samples were measured on the day of preparation and the results designated as day zero (d0) reference. Thereafter, samples were split and one half of the samples were kept at room temperature and the other half at 4°C. All samples were measured daily in week 1 and then twice a week for another 3 more weeks on a LSRII.

### Time series measurement: blood donation and every day work-up

Heparinized whole blood of 5 study patients (3 allergic patients, 1 sensitized but non-allergic subjects and 1 non-allergic individuals) was stimulated (PBS, or allergen, 5,000 ng/ml) and prepared for measurement according to the published standard protocol (Schwager et al. [2017] J. Allerg. Clin Immunol. 140:1331-1338). Thereafter, the blood was split and half of it kept at room temperature and the other half at 4°C. Starting at day one after the blood donation, a portion of the remaining blood was stimulated, prepared and measured daily in week 1 and then twice a week for another 2 more weeks on a LSRII.

### Interlaboratory survey

To access the practicality of the method in daily praxis, an interlaboratory survey was initiated and 9 further sites in Germany were included (i.e. Luebeck, Rostock, Berlin, Springe, Mainz, Bonn, Heidelberg, Constance, Cologne). For the interlaboratory survey, blood from 5 volunteers was drawn, stimulated with PBS, anti-lgE or allergen (Bet v 1, Der p 2 or peanut oleosin mix, 5,000 ng/mL) and further prepared according to the standard protocol (Schwager et al. [2017] J. Allerg. Clin Immunol. 140:1331-1338). The only difference was that the 3 ab mixture was used instead of the 12 ab cocktail. After sample preparation, samples were split and delivered by Deutsche Post AG (Bonn, Germany) to the respective destination sites. Instruments at the destination sites were set up using MACSQuant^{®} Calibration Beads (Miltenyi Biotec, Bergisch Gladbach, Germany) and our defaults within the measured parameters (FSC, SSC, PE, PE-Cy7 and APC) with regard to MFIs of the beads within the fluorochrome channels before measurements. Sample measurements were performed between 3 to 7 days after blood work up. Data analysis was conducted in Borstel with a fixed quadrant for the CD203c vs. CD63 gating, and calculation of the activation status of the basophils using % CD63⁺ cells of all basophils.

### Statistics

Medians, means and standard deviations were calculated using MS Excel (Microsoft, Redmont, USA). Data analysis and statistics were conducted using GraphPad Prism software package (version 6, GraphPad Software, San Diego, CA). Comparison between basophil markers and flow cytometry instruments was performed using the Mann-Whitney U test. Calculation of the optimal CD63-cut-off value was done via a receiver-operating characteristics (ROC) analysis. Comparison of the activation levels and the total number of basophils, was performed using the Wilcoxon signed-rank test.

### Study approval

The study was approved by the local ethics committee of the University of Luebeck. All study participants gave written informed consent prior to inclusion in the study.

### Computational data analysis (auto-gating)

On the basis of our highly sensitive and specific 3-marker gating strategy shown in Fiugre 2A, a data-driven automatic analysis was developed using flow cytometry Bioconductor tools in R (flowCore and associated packages). First, doublet exclusion was performed using an automatic singlet gate based on the FSC-A versus FSC-H regression (Fig. 7I). Subsequently, the identification of the basophil population was achieved by using a 2D gating algorithm based on the expression-normalized sum of CD203c and FcεRIα parameters followed by automatic ellipsoid gating (Fig. 7II). Finally, the fraction of stimulated basophils was estimated. For this, the main (negative) population of the merged unstimulated PBS controls was identified. Based on the mean and the standard deviation of this population a preliminary threshold was estimated. To determine the final thresholds for individual samples, the negative population of each sample was estimated using the preliminary threshold. Based on the mean and the standard deviation of this population the final threshold was calculated (Fig. 7III). This two-step process was necessary, because individual samples were somewhat inconsistent with regard to the location of their non-simulated sub-population, therefore requiring individual adjustments to achieve reliable separation between stimulated and non-stimulated cells.

### Results

### Choice of markers for basophil identification

At first, it had to be evaluated which antibodies from the previous 12-marker strategy (Schwager et al. [2017] J. Allerg. Clin Immunol. 140:1331-1338) might be helpful in a minimalistic protocol to identify basophils. Here, it was rather focused on rather basophil specific markers, CD203c and FcεRIα, while markers for other cell types such as CD3, CD4 or HLA-DR were excluded. Moreover, a further requirement for the desired implementation of an automated computer-based data analysis was the use of stable basophil markers that are less prone to be influenced upon allergen stimulation, and thus allow for a good distinction between basophils and other blood cells. Therefore, the robustness of CD203c, FcεRIα and CCR3, a marker set already used in commercially available BAT kits as described in EP 2037268 B1, was also tested and used as a benchmark.

When following the common gating strategy (CCR3 vs. SSC), a discrete basophil population appeared for PBS (CCR^{high}/SSC^{low}). However, a markedly reduced CCR3 expression was observed upon stimulation of basophils with stimulants, leading to a shift of basophils towards the lymphocyte population (Fig. 1A; anti-lgE #1). This effect becomes even more apparent when looking at the mean fluorescence intensity (MFI) histograms of PBS, anti-lgE and fMLP (a stimulant which triggers IgE-independent activation) (Fig. 1B). Here, a perceptible shift to lower fluorescence intensities can be observed for anti-lgE and fMLP.

For a better comparison of the overall robustness of the basophil markers, CCR3, CD203c and FcεRIα, we investigated the relative MFI change of samples stimulated with anti-lgE or fMLP to PBS. As shown in Fig. 1C, a significant difference was only observed in samples stained for CCR3. The MFI of CD203c, an identification and activation marker, was increased, while the MFI of FcεRIα remained almost unchanged.

The stimulation-associated downregulation of CCR3 becomes particularly problematic in cases of insufficient erythrocyte lysis, as the remaining erythrocytes start to appear in the basophil gate, and thus aggravate the gating of a discrete basophil population (Fig. 1A, anti-lgE #2). To avoid any issues related to an incomplete blood lysis and to build upon more robust basophil markers for the targeted automation process, we fundamentally questioned protocols recommended/pursued in commercial BAT kits (Fig.2a), and aimed at establishing a new more robust gating strategy.

### Development of a robust two identification marker gating strategy

For a new protocol, two markers for the identification of basophils together with CD63 as their activation marker, all bound to very bright fluorochromes (CD203c-PE, FcεRIα-PE-Cy7 and CD63-APC), were chosen. Starting with a two-dimensional dot plot, it was possible to identify basophils using CD203c and FcεRIα (Fig. 2A, I), followed by a forward scatter (FSC) versus side scatter (SSC) dot plot to exclude potential debris, erythrocytes or dendritic cells, which would appear lower (e.g. debris) or higher (e.g. dendritic cells) in size and granularity (Fig. 2A II). State-of-the-art practice for analyzing flow cytometric data includes also a doublet exclusion, which has been addressed with an area versus height presentation of the FSC signal (Fig. 2A, III). To reduce contaminations, another dot plot using the SSC (x-axis) versus FcεRIα (y-axis) presentation has been included, to eliminate dendritic cells by exclusion of their SSC higher and FcεRIα lower characteristics (Fig. 2A, IV). Finally, a CD203c versus CD63 dot plot was used to determine the percentage of activated basophils identified by the expression of CD63 (Fig. 2A, V). The quadrants and the cut off were set using PBS samples as negative controls. To verify the purity of the identified basophils, the contamination with cells being positive for CD3, CD14, CD16, CD19, CD20, CD56, and HLA-DR (Fig. 3A) was analyzed. Here, it has been found that the median contaminations were 0.1% (PBS), 0.2% (anti-lgE) and 0.5% (fMLP), respectively. To get information about the purity of the basophil population when using the commercially utilized CCR3 protocol in comparison to ours, the CCR3^{high}/SSC^{low} population for CD203c (Fig. 4) was analyzed. Although the blood lysis was successful, the CCR3⁺ gated basophil population showed a contamination with other cells. The median number of CD203c⁻ cells was 20% for PBS, 33% for anti-lgE and 28% for fMLP, reflecting a high contamination with non-basophil cells (Fig. 3B). It should be noted, that for these experiments, a CCR3 antibody with a much brighter fluorochrome compared to that exploited by kit manufacturers has been used (here: brilliant violet 421, BV421, which has a 3.6-fold higher stain index compared to R-phycoerythrin (PE)). This, in theory, should provide a better separation of individual cell populations.

To evaluate the reliability of our 3-marker gating strategy, the method according to the present invention has been compared to the high performance 12 ab protocol of Schwager et al. (Schwager et al. [2017] J. Allerg. Clin Immunol. 140:1331-1338). For this, 1,104 single samples (all stained with 12 ab) of 51 individuals (21 peanut-allergic patients, 15 peanut-sensitized subjects without clinical symptoms, and 15 non-allergic individuals) were analyzed with regard to the total number of basophils and their activation status using both protocols. These studies revealed no significant differences between the 12- and 3-marker tests with regard to the total number of basophils (Fig. 3C, allergic patients). More precisely, the median difference between samples was 0.5% (5% quantile: -10.3% and 95% quantile: 9.9%; data not shown). When comparing the basophil activation, we found almost no differences between both strategies as well, as indicated by the high correlation coefficient (R2 = 0.9965, see Fig. 3D) and a median difference of 0 (5% quantile: -1.56 percentage points (pps) and 95% quantile: 1.82 pps; data not shown). Consequently, the 3 ab gating protocol showed the same high diagnostic sensitivity and specificity as the 12 ab gating protocol, and thus was able to classify allergic and healthy individuals (peanut sensitized but non-allergic and non-allergic subjects) correctly. However, a huge difference was observed when looking at the costs for antibodies. Up to 86% of the expenses (calculated using list prices) can be saved when only 3 antibodies are being used instead of the 12 ab protocol of basophil gating.

### Elongated time frame for measurements

A current drawback of the BAT is that samples have to be analyzed within 24 hours after blood donation, making it very difficult to implement the BAT as routine diagnostic tool in centralized medical care units. As first data indicated that short-term storage of blood did not compromise subsequent basophil activation, we wanted to know if blood or prepared samples might be also storable for longer periods of time without affecting/compromising the outcome.

To analyze the reactivity of basophils in prepared samples over time, blood samples of 16 individuals (10 allergic patients, 3 sensitized but non-allergic individuals and 3 non-allergic subjects) were taken, stimulated (PBS, allergen and anti-lgE) once at day 0, and analyzed over a period of 4 weeks, for at least twice a week (see Fig. 5). To figure out whether storage conditions have an influence on the results, the initially prepared samples were split and stored either at 4°C or room temperature (RT, 19°C) under exclusion of light to prevent fluorochrome degradation. Here, it was found that the activation status of prepared samples did change only slightly over the course of 4 weeks when stored at 4°C (Fig. 5A), whereas it changed dramatically for samples which had been stored at room temperature. When looking at the 4°C-samples of allergic individuals, the median increase of the basophil activation in the presence of allergen was below 5 pps over the entire period of 28 days. In contrast, for the same samples stored at RT, a continuous rise of the basophil activation was observed over the first days, reaching 32 pps at day 14. With regard to the group of non-allergic subjects and sensitized individuals, a slight increase (mean 1.4 pps after 28 days) in basophil activation was observed for allergen-stimulated samples stored at 4°C, while for samples stored at RT, a steady increase for allergen-stimulated samples could be seen starting at day 4 after stimulation (mean 6.3 pps after 14 days). The background signal (PBS control) was unaltered in the samples stored at 4°C and increased only slightly over time for samples stored at RT. Storage at room temperature was accompanied by a continuous dying/fading out of the basophil population, eventually requiring to abort the measurements of these samples after day 14.

After having obtained these results, the inventors wanted to know whether donated blood could also be stored over a longer period of time without affecting the classification of the tested individuals. Therefore, blood of 5 individuals was sampled (3 allergic patients, 1 non-allergic subject and 1 sensitized but non-allergic individual), prepared and analyzed multiple times over a period of 3 weeks. To study the impact of the storage temperature, the initially donated blood samples were split into two parts which were stored analogously to the prior experiment (4°C and RT, respectively). With regard to the three allergic patients, a divergent picture for the basophil activation was observed over time. However, a reliable diagnosis of allergic patients was possible within the first 17 days, when the blood had been stored in the refrigerator. In fact, no differences in the activation levels of the basophils were observed for the control individuals. As for the previous experiment, it was required to discontinue the measurements of blood that has been stored at RT after 14 d, given the fast decline of viable basophils that could respond to the allergen stimulation.

To verify the observations, a field experiment in which the basophil activation of 3 allergic blood donors was investigated prior and after postal shipment was initiated (Figure 5C). Shipped samples were handed over to 5 different local post offices and analyzed as quintuple determinations 3 days after initial blood donation. As in the first experiments, slight disparities in the basophil activation level between fresh and stored samples were observed. However, the background signal (PBS) was still very low, and basophil activation induced by the allergen was significantly above background, meaning that according to the present invention, study participants could still be correctly identified as allergic patients after postal shipment of samples, as well as several days after sample preparation. Hence, these results verify that, using the 3-marker gating strategy according to the present invention, it possible to ship blood samples to other laboratories for BAT, with reproducible results.

### Interlaboratory testing (ring trial)

The interesting finding that the basophil activation can be measured even after several days after sample preparation, motivated the inventors to initiate a nationwide ring trial to verify their findings on an elongated time frame. Moreover, it was of interest to assess the robustness of the 3-marker protocol according to the present invention in cooperation with other laboratories (9) being equipped with different (6) flow cytometers and using different laser settings as well as slightly different filters for fluorochrome detection. For this purpose, blood of 5 allergic individuals was collected, prepared and stimulated with allergens (1x Bet v 1, 3x Der p 2, 1x peanut oleosins), anti-lgE and PBS. Thereafter, the samples were divided and sent via conventional mail to the different core facilities in Lübeck, Rostock, Berlin, Springe, Constance, Heidelberg, Mainz, Bonn and Cologne. A protocol and some calibration beads to set up the different instruments, using defined mean values in the detectors for FSC, SSC, PE, PE-Cy7 and APC were provided together with the samples. Additionally, four thresholds (FSC, SSC, PE, PE-Cy7) had to be set, as well as a stopping gate on basophils (800 events). Only the FACSCalibur (Springe) was limited to 2 thresholds (FSC, PE-Cy7), because of the technical specifications of the instrument. Furthermore, samples in Heidelberg were measured on two different flow cytometers. Overall, the desired number of basophils was recorded, and activation of basophils was detectable at all facilities. With regard to the results, only slight differences for the basophil activation were observed when comparing our data with those of our cooperation partners. The mean difference for samples incubated with PBS, anti-lgE and allergen were 0.43 percentage points (pps) (range 0.01 to 2.33 pps), 0.63 pps (range 0.01 to 5.28 pps) and 1.60 pps (range 0.05 to 11.07 pps), respectively. Moreover, a generally very low background (mean 0.28%) was observed for all samples at all sites.

### Automatic sample processing and measurement to reduce hands-on time

Although the BAT is superior to other *in-vitro* allergy tests, its integration into routine diagnostic is largely hampered by the enormous laboratory work, as all commercially available BAT kits need to be performed in tubes due to large volume of added reagents (>2 ml). As this approach impedes a high-throughput measurement of samples, it was tried to transfer the 3 ab protocol into an automated 96-well plate format. Here, we established an automatic sample processing and measurement using a flow cytometer with integrated robotic functions (MACSQuant10, Miltenyi Biotec GmbH, Bergisch Gladbach, Germany). After having manually performed the incubation of blood and stimulants in a 96-deepwell plate, the plate was transferred to the 96-well plate holder at the instrument. Here, the automated protocol, which includes addition of antibodies and blood lysis, dilution of samples, incubation of samples and sample measurement, was conducted on a MACSQuant10 (MQ10). To assess the feasibility of this approach, the manual sample preparation of Schwager et al. (Schwager et al. [2017] J. Allerg. Clin Immunol. 140:1331-1338), was compared to the automated work-up using the same samples in parallel (Fig. 6). Although measurements were conducted on two different flow cytometers (manual: LSRII; automated: MQ10, according to our SOP's), both methods showed very similar basophil activation levels for the different allergens and controls used. However, the automated protocol largely reduced the average hands-on time for 96 samples from about 9 hours (manual work) to 1.5 hours (automation). In addition, the required working time for the data analysis was omitted, thus reducing the overall hands-on time to approximately 30 minutes. When only taking into account the 21 allergic individuals, which were tested for up to 22 allergens at (at least) four concentrations, the automated work-up saves approx. 9,450 minutes (157.5 h) of working time.

### Development of a robust automated analysis template using R Studio

Based on 1,389 individual BAT samples, a data-driven automatic analysis algorithm has been created using Bioconductor tools in R. The performance of this algorithm was validated by comparing the results obtained to the results of the same data set analyzed manually by an expert. During the evaluation of the algorithm, it was found that basophil activation levels of samples with very small numbers of basophils differed significantly between both methods. Accordingly, although the implemented auto-gating does not require a minimum number of basophils for the correct setting of gates, a threshold of 100 basophils was set as minimum requirement for the analysis by the algorithm. With this threshold, 1,376 of 1,389 samples (99.1%) could be analyzed automatically. Here, the comparison of the automatic assessment to the manual evaluation showed good agreement with respect to the activation level and the total number of total basophils, both resulting in high correlation coefficients of 0.952 and 0.967 (P <0.0001), respectively. With respect to the clinical data, the automatic analysis resulted in the same diagnostic sensitivity (100%), but a slightly lower specificity (97%) for peanut oleosins compared to the previously published results of Schwager et al. Only one peanut-sensitized individual was misclassified due to an exaggeration of the activated proportion of basophils by the algorithm. However, in most cases, the results of the algorithm were almost identical to that of the manual gating conducted by an expert. For a more convenient use in daily routine practice, we also implemented a visual quality control and quality checks to identify technical errors and problems derived from the sample preparation. The program displays a warning in cases where (i) the total number of basophils is below 100, (ii) the basophil fraction is below 0.1% or above 2%, (iii) total events are below 10,000, (iv) the singlet fraction is below 80% (scatter problems) and/or (v) if there is a large unusual spread of the basophil population.

### Discussion

The dramatic increase of allergic diseases, particularly in Western countries, has led to a growing demand of reliable diagnostic tests for the definite proof of an allergy. Unlike the SPT and the IgE-antibody detection tests, which only confirm a sensitization to an allergen, the basophil activation test (BAT) reveals the biological consequences of the existing IgE antibodies by studying the behavior of the basophils towards the applied stimulants. However, there are many BAT protocols with different identification markers, often claiming to be robust even with only a single surface marker exploited. For example, the basophil identification marker CCR3 is already in use in commercially available BAT kits, and its application as single basophil marker can be sufficient for non-stimulated samples. Nonetheless, due to the stimulation-induced decrease of the CCR3 signal, the basophil population tends to shift towards the CCR3 negative leucocyte population, which leads to the risk of a considerable contamination of the basophils. As a consequence, the basophil activation level may be underestimated. This is particularly the case for samples that have been stimulated with allergens - which are of course the most relevant samples with regard to the diagnostic outcome for the patient. Therefore, the single use of CCR3 has been reported to be insufficient, and also its designation as a robust marker is still a matter of debate (Chirumbolo et al. [2012] J. Internat. Soc. Analyt. Cytol. 2011;79:102-106.).

In contrast to the single use of CCR3, the protocol according to the present invention which uses a combination of CD203c and FcεRIα is a more robust approach that in combination with our new gating strategy allows for the identification of a pure basophil population. Using the identification marker (CD203c) - that is upregulated by stimulants rather than downregulated - seems to be beneficial as basophils are shifted away from any contamination.

From the technical side, the protocol according to the present invention includes doublet exclusion (exclusion of cells sticking together), a today's state-of-the-art technique that is, however, not used by any of the commercially available BAT kits. Moreover, very bright fluorochromes with an optimized low spillover to facilitate an optimized gating and analysis of basophils are used. For identification of basophils, a PE labelled anti-CD203c ab and a PE-Cy7 labelled anti-FcεRIα ab are used. An allophycocyanin (APC) labelled anti-CD63 ab is used to assess the basophils' activation status. According to the Boston University Flow Cytometry Core Facility, PE and APC are classified as very bright fluorochromes (class 5) and PE-Cy7 as bright (class 4). Although one has to be aware of potential spillover effects caused by the combination PE/PE-Cy7, both fluorochromes were chosen because the majority of flow cytometers can be addressed with our new protocol as they operate with the two lasers (488nm and 633nm) required. Indeed, the spillover effects observed were minimal and did neither influence the analysis nor the outcome of measurements, as CD203c was only used as identification marker, but not as activation marker. Therefore, there was no need for any compensation, which normally has to be done carefully.

To evaluate the performance of the 3 ab gating protocol according to the present invention, a data set of more than 1,100 samples (51 study patients) was re-analyzed and compared to the results of the highly specific and sensitive 12 ab gating protocol, described by Schwager et al., see above. This comparison revealed that the number of identified basophils and their activation level were almost identical with a median difference in identified basophils of 0.5% and a median difference of zero for the activation level. A re-analysis of the published receiver-operating characteristic of Schwager et al., with the data of the 3 ab protocol according to the present invention also showed identical results, namely a diagnostic sensitivity and specificity of 100%. Here, it is of note that these results were achieved without the use of any extrinsic stimulants such as IL-3. Although many protocols rely on the addition of IL-3, whose use is controversially discussed, and the European Academy of Allergy and Clinical Immunology (EAACI) even suggests avoiding any *in-vitro* stimulants. In conclusion, the 3 ab protocol established represents a powerful approach to dramatically reduce complexity and costs (-86%) of the BAT without affecting its superior diagnostic performance.

A major hurdle for the implementation of the BAT into routine diagnostic laboratories is the requirement to analyze blood samples within a few hours after donation. Recent results indicated, however, that a delayed analysis of samples even after 5 days of storage may be feasible. Accordingly two independent time series, one evaluating the storage of prepared samples (TS1) and one of drawn blood (TS2), have been set up and studied. For TS1, studying samples derived from ten allergic patients and six controls, it was found that the median change in the activation of basophils stimulated by an allergen remained within a 5 pps interval over a period of 28 days when samples were stored at 4°C. In contrast, allergen-stimulated samples stored at RT crossed the 5% boundary at day 5 (allergic patients) and day 10 (controls), respectively. This aspect is particularly important for the controls as this issue bears the risk of false positive results, and thus for misclassification. However, the results of TS1 show that the measurement of stored samples after stimulation, staining and preparation is possible for a couple of days and up to at least 4 weeks, depending on the storage conditions. Moreover, the high diagnostic performance of the BAT can be maintained even under conditions of elevated basophil activation, by setting the cut-off for positivity to higher values as the mean basophil activation is increased for both groups. From the experimental side, for both storage conditions, a decrease of the basophil fraction within the FSC/SSC was observed over time, whereas the loss of viable cells was connected to prolonged acquisition times at the flow cytometer as well as formation of white debris in the storage tube.

The investigation of the basophil reactivity upon long-term storage of blood (TS2) revealed that sample preparation and flow cytometric measurement do not necessarily have to be conducted within 24 h. In fact, correct classification of the included subjects was still possible after 7 days of storage at RT, or after 17 days of storage at 4°C, although in both cases a decrease in basophil reactivity could be observed for 2 of 3 allergic individuals. Furthermore, an increased viscosity of the blood and a drastic reduction of cells were observed, particular for samples stored at RT. These observations prompted us to start a real-world pilot study to evaluate the possibility of shipping blood samples prior to BAT analysis. The corresponding data show the same tendencies as in the blood storage experiments, namely a small alteration in the basophil activation level. This issue might be (in parts) attributed to the daily preparation of stimulants and buffers, which of course underlay unavoidable variations, e.g. pipette inaccuracies that can impact the obtained results. In general, the results open up new dimensions of cooperative studies for scientists and clinicians without access to flow cytometers and also for routine laboratories.

With the increased need for reliable allergy diagnostic tests, there is also a rising awareness of commercial entities to provide robust assays for the screening of potentially allergic patients. For this reason, we initiated a nationwide ring trial to evaluate the robustness of our protocol and to verify the practical usefulness of our findings on the fixation of prepared samples. The operators of the different centers were supplied with calibration beads for the instruments and encouraged to follow our standard operation procedures in order to minimize user-based as well as technical variations. Overall, only small differences were observed between the participating institutions resulting in an inter-assay CV of 7.82% for IgE and 6.71% for the allergens.

Although we have shown a solution for the logistical restrictions of the BAT, its routine application in medical care units and analytical laboratories is largely hampered by the time-consuming and laborious handling of samples in single tubes that is mandatory when using commercial BAT kits. To address this limitation, the antibody staining panel was transferred to a flow cytometer with integrated robotic functions. Here, only the preparation of stimuli and the addition of blood have to be done manually. All other steps, including erythrocyte lysis, antibody staining, washing and measurement are fully automated. This is the first approach to realize a high-through-put BAT analysis. In a direct comparison with the manual protocol, we observed no differences in the activation levels of the basophils. However, the automated sample processing was able to save more than 80% of the working time.

In addition, if also a subsequent automated data analysis template was used, the analysis time of approximately 1 h (about 15% of the total time) could be further reduced to the time required for the transfer of the FCS files to the analysis server. Ideally, such an algorithm could be integrated in the instruments' software in the future to provide an all in one solution for operators. In general, an algorithm offers transparency, reproducibility, efficiency, quality control and multiple aggregated statistics of basophil activation. The first (and so far only) BAT data-driven algorithm was published by Patil et al. (Patil et al. [2018] Cytom. B. Clin. Cytom. 94:667-673). Although their approach was promising, the basis for the data acquisition was a CCR3-based BAT kit, which - as shown above - is less robust, compared to the protocol according to the present invention. This might be one reason for their higher number of experiments that required manual gating (8.5% vs. 0.9%) due to poor basophil identification achieved by the Patil algorithm. For this reason, the use of robust basophil markers is part of essence and a prerequisite to increase the success of data-driven algorithms, given that the accuracy in setting gates stands and falls with the total number of basophils.

## Claims

1. A cytometric based *in vitro* method for the determination of basophil activation in a sample obtained from a subject comprising
a. providing a sample of said subject;
b. optionally, treating/stimulating the sample with a test substance;
c. adding a mixture of antibodies each labelled with a different fluorochrome, said mixture containing the labelled antibodies anti-CD63, anti-CD203c and anti-FcεRIα, and incubating said mixture with said sample;
d. measuring the labelled sample obtained in step b) by flow cytometric based measurement;
e. determining basophil activation based on the analysis of the measured samples and obtained data by flow cytometry based measurement whereby the analysis is based on the following:
i. excluding doublets, in particular by of analyzing forward scatter area versus forward scatter height, and gating of single cells;
ii. analyzing the cells gated in i) and gating basophils being double positive for Fc_{ε}RI_{α} and CD203c;
iii. analyzing the basophils gated in ii) on forward scatter area versus side scatter area and gating of basophils;
iv. analyzing the basophils selected in iii) for expression of CD203c versus expression of CD63 and determining the percentage of basophils expressing both CD203c and CD63 representing activated basophils.

2. The method according to claim 1 wherein the sample is selected from fluid or enzymatically digested tissue for single cell suspensions of said subject, in particular, selected from sputum, blood, including whole blood, saliva, nasal secretion, and urine, preferably the sample is whole blood.

3. The method according to any one of the preceding claims wherein the method is a method for the determination of basophil activation induced by a test substance.

4. The method according to claim 3 wherein the test substance is a mitogen, an antigen, an allergen, a protein or peptide, like a protein or peptide allergen, a group or mixture of protein and/or peptide allergens, a non-proteinaceous allergen, a low molecular weight allergen, a low molecular weight drug substance or a hapten.

5. The method according to claim 3 or 4 wherein the test substance is an allergen, in particular, selected from the group of inhalant allergens, food allergens, insect allergens and pharmaceutical based allergens, including peanut allergens, like peanut oleosins, peanut defensins, Der p 2, Bet v 1, Ara h 1, Ara h 2, Ara h 6, Ara h 8, Ara h 14, Ara h 15.

6. The method according to any one of the preceding claims wherein the sample is whole blood or purified whole blood comprising anti-coagulation compounds like EDTA, citrate, heparin as anticoagulant and the method includes lysing erythrocytes present in said sample.

7. The method according to any one of the preceding claims wherein flow cytometry is conducted using at least two lasers for excitation of the fluorochrome labelled antibodies, in particular, a first laser for forward and side scatter measurement as well as the identification of the basophils (like Fc_{ε}RI_{α} and CD203c) and a second laser for the measurement of the basophil activation status (like CD63).

8. The method according to any one of the preceding claims wherein the fluorochromes are selected for having low spill over, in particular, the fluorochromes used with the first laser are PE, PE-Cy7, and allophycocyanine (APC) with the second laser, respectively.

9. The method according to any one of the preceding claims wherein the step e) is conducted by a computer implemented analysis method.

10. A computer implemented method for the determination of basophil activation in samples obtained from a subject comprising the steps of:
a. obtaining data of measured parameters by flow cytometry including the following: forward scatter area, forward scatter height, side scatter area, fluorescence of the labelled, basophil-bound antibody anti-CD203c, fluorescence of the labelled, basophil-bound antibody anti- Fc_{ε}RI_{α}, fluorescence of the labelled, basophil-bound antibody anti-CD63 in an stimulated or non-stimulated sample of a subject;
b. computing the data obtained as follows:
i. doublet exclusion, in particular, based on forward scatter area versus forward scatter height;
ii. analyzing the cells gated in i) and gating basophils being double positive for Fc_{ε}RI_{α} and CD203c;
iii. analyzing the basophils gated in ii) on forward scatter area versus side scatter area and gating of basophils;
iv. analyzing the basophils selected in iii) for expression of CD203c versus expression of CD63 and determining the percentage of basophils expressing both CD203c and CD63, representing activated basophils;
c. identifying the percentage or absolute number of basophil activation in said sample of said subject;
d. optionally further comprising the step of showing the data on an output unit, in particular, showing the percentage of basophil activation;
e. optionally, quality control of the samples based on at least one of i) minimum amount of basophils to be analyzed, ii) minimum background activation of PBS samples (like below 5%), iii) minimum amount of all events (like above 10,000, and iv) maximum amount of doublets (like below 20% of all events).

11. The computer implemented method according to claim 10 for determination of basophil activation induced by a test substance in a sample of a subject, in particular, wherein the test substance is an allergen.

12. Computer readable medium or computer program product having computer executable instructions for performing the steps as identified in any one of the claims 10 to 11.

13. A test kit or kit of parts for determining of basophil activation in a subject, said kit comprising anti-CD203c, anti-FcεRIα and anti-CD63 antibodies, each labelled with a distinct fluorochrome; optionally lysis buffer for erythrocytes and, optionally, washing buffer, and instructions on how to use said test kit or kit of parts in a method according to any one of the claims 1 to 11.

14. The use of the test kit, or the use of a kit of parts according to claim 13 for the determination of basophil activation by flow cytometric measurement, in particular, basophil activation induced by a test substance *in vitro.*

15. The use of a test kit, or the use of a kit of parts according to claim 13 for the determination in a subject of i) having allergy against a test substance being an allergen based on determination of basophil activation or ii) suffering from an autoimmune disease.

16. A method according to any of the preceding 1 to 11, wherein the sample processing and measurement are conducted automatically using a flow cytometer with integrated robotic sample handling and manipulation, and wherein the basophil activation is optionally determined using a computer-implemented method according to any of the claims 10 and 11.

17. A method according to any of the preceding claims 1 to 11 or 16, wherein the blood sample to be analyzed has been stored at room temperature for up to 7 days or at 4°C for up to 21 days before stimulation, processing and measurement, or alternatively, wherein the stimulated sample has been stored in the dark at 4°C for up to 28 days before determination of the basophil activation.
